# EUROPÄISCHE PATENTSCHRIFT

(11) **EP 1 874 943 B1**
(45) Veröffentlichungstag und Bekanntmachung des Hinweises auf die Patenterteilung: **05.08.2009**
(21) Anmeldenummer: 06754774.5
(22) Anmeldetag: 20.04.2006
(51) Int. Cl.: C12P 13/02

(54) **ENZYMATISCHE SYNTHESE VON POLY(OXYALKYLEN)ACRYLAMIDEN**
ENZYMATIC SYNTHESIS OF POLY(OXYALKYLENE)ACRYLAMIDES
SYNTHESE ENZYMATIQUE DE POLY(OXYALKYLENE)ACRYLAMIDES

(30) Priorität: 22.04.2005 DE 102005018935
(43) Veröffentlichungstag der Anmeldung: 09.01.2008
(73) Patentinhaber: BASF SE, 67056 Ludwigshafen (DE)
(72) Erfinder: HÄRING, Dietmar, 69198 Schriesheim (DE); HAUER, Bernhard, 67136 Fussgönheim (DE); BECKER, Stefan, 68167 Mannheim (DE)
(86) Internationale Anmeldenummer: PCT/EP2006/061716
(87) Internationale Veröffentlichungsnummer: WO 2006/111566

(56) Entgegenhaltungen:
- DE-A1- 3 130 508
- US-A- 5 567 422
- US-A- 5 973 203

## Beschreibung

Die Erfindung betrifft ein Verfahren zur enzymatischen Synthese von Poly(oxyalkylen)acrylamiden.

### Stand der Technik:

Die Poly(oxyalkylen)acrylamide sind auf verschiedenen Wegen zugänglich. Die chemische Synthese von Poly(oxyalkylen)acrylamiden erfolgt durch direkte Ver- oder Umesterung von Acrylsäure oder Acrylsäureestern mit Poly(oxyalkylen)aminen und verläuft bei Temperaturen von über 100°C unter Säurekatalyse (DE 3130508 Röhm GmbH 1981).

Wegen der hohen Temperaturen ist der Zusatz großer Mengen von Polymerisationsinhibitoren notwendig. Durch die hohen Reaktionstemperaturen entstehen Michael-Addukte des Amins an die Doppelbindung der Acrylsäureverbindung. Es entstehen komplexe und oft dunkle Produktgemische mit störendem Geruch. Die Reaktion von Acryloylchlorid mit Aminen ergibt ebenfalls N-Alkyl-acrylamide. Das Verfahren ist aber wegen der hohen Kosten für Acryloylchlorid nicht wirtschaftlich.

Die biokatalytische Synthese von Acrylsäureamiden ist in den folgenden Dokumenten beschrieben. Puertas et al. (Tetrahedron 1993, 49, 4007-4014) beschreiben die Reaktion von Methyl(meth)acrylat mit Butylamin, Benzylamin, 2-Aminobutan und 2-Aminoheptan. Die Lipase-katalysierte Synthesen ergaben innerhalb von 3 -10 Tagen Ausbeuten von 40-95%.

Sanchez et al. (Synlett 1994, 529-530) beschreiben die Ammonolyse von Methyl-(meth)acrylat mit Ammoniak zu (Meth)acrylamid. Die Lipase-katalysierte Reaktion ergab bis zu 91 % Ausbeute nach 93 h.

Margolin et al. (J. Am. Chem. Soc. 1991, 113, 4693-4694) beschreiben die Reaktion von Trifluoroethylmethacrylat mit 1-(1-Naphtyl)ethylamin oder Phenylalaninamid. Die Reaktion wurde durch die Protease Subtilisin katalysiert.

Egraz et al. (US 5,973,203) beanspruchen die enzymatische Synthese von (Meth)Acrylamiden enthaltend tertiäre Aminogruppen. Dabei reagiert ein (Meth)Acrylsäurealkylester mit einem Diamin, das eine primäre und eine tertäre Aminogruppe hat.

### Kurze Beschreibung der Erfindung

Aufgabe der Erfindung war es daher, ein Verfahren zur Herstellung von Poly(oxyalkylen)acrylamid zu entwickeln. Die Synthese sollte insbesondere die Darstellung von Poly(oxyalkylen)acrylamiden in hoher Reinheit und guter Ausbeute ermöglichen und das Auftreten von unerwünschten Nebenreaktionen unterdrücken.

### Detaillierte Beschreibung der Erfindung

Ein erster Gegenstand der Erfindung betrifft ein Verfahren zur enzymatischen Synthese von Poly(oxyalkylen)acrylamid, wobei man ein aliphatisches Poly(oxyalkylen)amin in Gegenwart einer Hydrolase in Substanz oder in einem ein organisches Lösungsmittel umfassenden, flüssigen Reaktionsmedium mit einer Acrylsäureverbindung oder einem Alkylester davon umsetzt, und man das (die) gebildete(n) Poly(oxyalkylen)acrylamid nach Beendigung der Reaktion gegebenenfalls aus dem Reaktionsgemisch isoliert.

Ein "aliphatisches Poly(oxyalkylen)acrylamid" in Sinne der Erfindung ist ein- oder mehrfach acryliert.

Der erfindungsgemäß erzielte Umsatz (molarer Anteil an Poly(oxyalkylen)acrylamid, die mindestens eine Amidgruppe tragen) liegt erfindungsgemäß bei mindestens 20 Mol-%, wie z. B. 20 bis 100 Mol-%, 40 bis 99 Mol-%, 50 bis 95 Mol-% oder 75 bis 95 Mol-%, jeweils bezogen auf die eingesetzten Mole Poly(oxyalkylen)amin.

Das flüssige organische Reaktionsmedium kann einen anfänglichen Wassergehalt von bis zu etwa 10 Vol.-% aufweisen, ist vorzugsweise aber im wesentlichen wasserfrei. Die Reaktion kann dabei in Substanz oder falls vorteilhaft auch nach Zugabe eines geeigneten organischen Lösungsmittels erfolgen.

Als organische Lösungsmittel finden vorzugsweise solche Verwendung, die ausgewählt sind unter tert.- Butanol und tert.-Amylalkohol, Pyridin, Poly-C₁-C₄-alkylenglykoldi-C₁-C₄-alkylethern, insbesondere Polyethylenglycoldi-C₁-C₄-alkylether, wie z.B. Dimethoxyeethan, Diethylenglycoldimethylether, Polyethylenglycoldimethylether 500, tert.-Butylessigsäureester, MTBE, Aceton, 1,4-Dioxan, 1,3-Dioxolan, THF, Dimethoxymethan, Dimethoxyethan, Cyclohexan, Methylcyclohexan, Toluol, Hexan sowie deren ein- oder mehrphasigen Mischungen.

Im erfindungsgemäßen Verfahren werden Acrylsäureverbindung und Poly(oxyalkylen)-amin im allgemeinen in einem molaren Verhältnis von etwa 100:1 bis 1:1, wie z.B. im Bereich von 30:1 bis 3:1 oder 10:1 bis 5:1, eingesetzt.

Die anfängliche Poly(oxyalkylen)amin- Konzentration liegt z.B. im Bereich von etwa 0,1 bis 20 Mol/l, insbesondere 0,15 bis 10 Mol/l.

Vorzugsweise ist das Poly(oxyalkylen)amin ausgewählt unter Polyoxyalkylenaminen , bevorzugt Polyoxyethylenaminen und Polyoxypropylenaminen oder gemischten Polyoxyethylen-propylenaminen, mit 1 - 250, bevorzugt 2 - 100, besonders bevorzugt 3 - 50 Oxyalkyleneinheiten und mindestens einer, bevorzugt genau einer terminalen Aminofunktion.

Die Poly(oxyalkylen)amin ("Polyetheramine") werden durch Alkoxylierung von Alkoholen und nachfolgender Aminierung mit Ammoniak hergestellt, wie es beispielsweise in WO 01/98388 beschrieben wird. Sie sind auch kommerziell erhältlich, z.B. von BASF oder Huntsman ("Jeffamine"^{®}).

Die erfindungsgemäß eingesetzte "Acrylsäureverbindung" ist vorzugsweise ausgewählt unter Acrylsäure, Methacrylsäure, deren Anhydride, Niedrigalkyl- d.h. C₁-C₆-Alkyl-substituierter (Meth)Acrylsäure, den C₁-C₂₀-Alkylestern davon oder Ethylenglykoldiacrylaten; sowie Mischungen dieser Verbindungen. Bevorzugte C₁-C₆-Alkyl-Gruppen sind insbesondere Methyl- oder Ethylgruppen. Bevorzugte C₁-C₂₀-Alkyl- Gruppen sind z.B. Methyl, Ethyl, i- oder n-Propyl, n-, i-, sec.- oder tert.-Butyl, n- oder i-Pentyl; außerdem n-Hexyl, n-Heptyl, n-Octyl, n-Nonyl, n-Decyl, n-Undecyl, n-Tridecyl, n-Tetradecyl, n-Pentadecyl und n-Hexadecyl, und n-Octadecyl, sowie die ein oder mehrfach verzweigten Analoga davon. Bevorzugt verwendet man (Meth)Acrylsäure oder (Meth)Acrylsäurederivate.

Geeignete Derivate obiger Acrylsäureverbindungen, wie z.B. Acrylsäure und (Meth)Acrylsäure, sind Ester mit gesättigten und ungesättigten, cyclischen oder offenkettigen C₁-C₁₀-Monoalkoholen, insbesondere die Methyl-, Ethyl-, Butyl- und 2-Ethylhexyl ester. davon. Die erfindungsgemäßen C₁-C₁₀-Monoalkohole umfassen bevorzugt C₁-C₆-Alkylgruppen obiger Definition oder deren längerkettigen, gegebenenfalls verzweigten, Homologen mit bis zu 10 Kohlenstoffatomen oder C₄-C₆-Cycloalkyl Gruppen, wie Cyclopropyl, Cyclopentyl oder Cyclohexyl, welche gegebenenfalls durch eine oder mehrere Alkylgruppen mit 1 bis 3 C-Atomen substituiert sein können.

Werden keine anderen Angaben gemacht so steht erfindungsgemäß C₁-C₆-Alkyl für Methyl, Ethyl, n- oder i-Propyl, n-, sec.- oder tert.-Butyl; n- oder tert.-Amyl, sowie geradkettiges oder verzweigtes Hexyl. C₃-C₆-Alkyl steht insbesondere für n- oder i-Propyl, n-, sec.- oder tert.-Butyl, n- oder tert.-Amyl, sowie geradkettiges oder verzweigtes Hexyl. C₁-C₄-Alkylen steht vorzugsweise für Methylen, Ethylen, Propylen oder 1- oder 2-Butylen.

Die erfindungsgemäß eingesetzten Hydrolasen sind ausgewählt unter Esterasen (E.C. 3.1.-.-), wie insbesondere Lipasen (E.C. 3.1.1.3), Glykosylasen (E.C. 32.-.-) und Proteasen (E.C. 3.4.-.-) in freier oder immobilisierter Form. Besonders geeignet sind Novozyme 435 (Lipase aus Candida antartica B) oder Lipase aus Aspergillus sp., Burkholderia sp., Candida sp., Pseudomonas sp., oder Schweinepankreas.

Der Enzymgehalt im Reaktionsmedium liegt insbesondere im Bereich von etwa 0,1 bis 10 Gew.%, bezogen auf das eingesetzte Polyol. Die Enzyme können in der erfindungsgemäßen Umsetzung in reiner Form oder geträgert (immobilisiert) eingesetzt werden.

Das erfindungsgemäße Verfahren wird vorzugsweise so durchgeführt, dass die Reaktionstemperatur im Bereich von 0 bis etwa 100°C wie insbesondere im Bereich von 20 bis 80°C liegt. Die Reaktionsdauer liegt gewöhnlich im Bereich von etwa 1 bis 72, bevorzugt 6 - 24 Stunden.

Der gegebenenfalls bei der Reaktion anfallende Alkohol (in der Regel ein einwertiger Alkohol, wie z.B. Methanol oder Ethanol) oder das bei der Amidierung anfallende Reaktionswasser kann, falls erforderlich, in geeigneter Weise aus dem Reaktionsgleichgewicht, kontinuierlich oder schrittweise, entfernt werden. Hierzu eignen sich vorzugsweise Molekularsiebe (Porengröße z.B. im Bereich von etwa 3 - 10 Angström), oder eine Abtrennung durch Destillation, durch geeignete semipermeable Membranen oder durch Pervaporation.

Zur Durchmischung des Reaktionsansatzes können beliebige Verfahren eingesetzt werden. Spezielle Rührvorrichtungen sind nicht erforderlich. Das Reaktionsmedium kann ein- oder mehrphasig sein und die Reaktanden werden darin gelöst, suspendiert oder emulgiert, gegebenenfalls zusammen mit dem Molekularsieb vorgelegt. Zum Start der Reaktion kann das Medium mit dem Enzympräparat versetzt werden. Die Temperatur wird während der Reaktion auf den gewünschten Wert eingestellt.

Alternativ kann die Reaktion auch so durchgeführt werden, dass das Enzym in immobilisierter Form in einem Festbettreaktor vorgelegt und der Reaktionsansatz über das immobilisierte Enzym, gegebenenfalls im Kreislauf, gepumpt wird. Reaktionswasser und/oder -alkohol können dabei ebenfalls kontinuierlich oder schrittweise aus dem Reaktionsgemisch entfernt werden.

Das erfindungsgemäße Verfahren kann diskontinuierlich, halbkontinuierlich oder kontinuierlich in herkömmlichen Reaktoren (z.B. Rührkessel, Festbettreaktoren) durchgeführt werden.

Nach Beendigung der Reaktion kann man das gewünschte Poly(oxyalkylen)acrylamid aus dem Reaktionsansatz isolieren, beispielsweise chromatographisch aufreinigen, und dann zur Herstellung der gewünschter Polymere oder Copolymere einsetzen. Die Reaktionsmischung kann auch nach Abtrennen des Enzyms (Filtration, Dekantieren) direkt weiterverwendet werden.

### Experimenteller Teil

Folgende Poly(oxyalkylen)amine wurden eingesetzt:
- "Polyetheramin 520": Polyethylenglykolamin-monomethylether (ca. 520 g/mol)
- "Polyetheramin 750": Polyethylenglykolamin-monomethylether (ca. 750 glmol)

### Beispiel 1: Herstellung von Poly(oxyalkylen)acrylamid (Variation des Reaktandenverhältnisses)

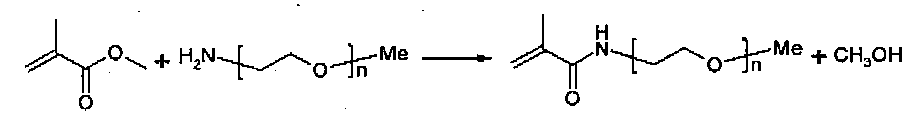

Es wurden 5 mmol Polyetheramin 750 (3,75 g), 25 oder 50 mmol Methylmethacrylat (MMA), ggf. 1,0 g Molsieb (5 A) und 200 mg Novozym 435 (geträgerte Lipase aus Candida antarctica der Firma Novozymes, Dänemark) während 24 h bei 60°C gerührt. Das Enzym wurde abfiltriert, mit etwas Methyl-tert-butylether nachgewaschen und der Überschuss MMA und MTBE am Rotationsverdampfer im Vakuum entfernt. Der Umsatz hing vom Überschuss Methylmethacrylat ab wie folgende Tabelle zeigt.

| molverhältnis | Umsatz [%] | Umsatz [%] |
|---|---|---|
| Amin / MMA | ohne Molsieb | mit Molsieb |
| 1/10 | 98 | 98 |
| 1/5 | 83 | 84 |

### Produktanalvtik

In der H-NMR-Analyse in CD₃OD₃ verschwindet das Signal des "Amin-Edukts" bei 2,75 ppm (CH₂-NH₂) und ein neues "Amid-Signal" bei 3,43 ppm (CONH-CH₂) entsteht. Der Umsatz wurde über die Integrale wie folgt berechnet: (Amid*100 %) *l* (Amid+Amin).

### Beispiel 2: (Zusatz von Methacrylsäure)

Es wurden 5 mmol Polyetheramin 520 (2,6 g), 50 mmol Methylmethacrylat (MMA), ggf. 1,0 g Molsieb (5 A), verschiedene Mengen Methacrylsäure (MAS) und 200 mg Novozym 435 (geträgerte Lipase aus Candida antarctica der Firma Novozymes, Dänemark) während 24 h bei 20°C gerührt. Das Enzym wurde abfiltriert, mit etwas Methyl-tert-butylether nachgewaschen und der Überschuss MMA und MTBE am Rotationsverdampfer im Vakuum entfernt. Der Umsatz hing vom Zusatz Methacrylsäure ab wie folgende Tabelle zeigt.

| Methacrylsäure | Umsatz [%] | Umsatz [%] |
|---|---|---|
| [mmol] | ohne Molsieb | mit Molsieb |
| 0 | 77 | 79 |
| 0,05 | 78 | 79 |
| 0,5 | 100 | 96 |

### Beispiel 3: (Präparativer Ansatz)

Es wurden 0,25 mol Polyetheramin 750 (187,5 g), 2,5 mol Methylmethacrylat (250 g) und 10,0 g Novozym 435 (geträgerte Lipase aus Candida antarctica der Firma Novozymes, Dänemark) während 24 h bei 60°C gerührt. Das Enzym wurde über eine Filternutsche abgesaugt, mit etwas Methyl-tert-butylether nachgewaschen und der Überschuss Methylmethacrylat und MTBE am Rotationsverdampfer im Vakuum entfernt. Man erhielt 183 g eines klaren, schwach gelblichen Öls, das ohne weitere Aufarbeitung polymerisiert werden konnte. Man erhielt einen Umsatz von >99 %.

### Beispiel 4: (Präparativer Ansatz)

Es wurden 52,0 g Polyetheramin 520 (0,1 mol), 100,1 g Methylmethacrylat (1,0 mol) und 4,0 g Novozym 435 für 24 h bei 40°C geführt Zur Aufarbeitung saugte man das geträgerte Enzym über eine Filternutsche ab, wusch mit etwas MTBE nach und entfernte überschüssige Lösemittel und MMA am Rotationsverdampfer im Vakuum. Man erhielt 55,6 g einer farblosen Flüssigkeit. Das Amin war laut H-NMR-Analyse zu 95 % mit MMA umgesetzt worden.

## Patentansprüche

1. Verfahren zur enzymatischen Synthese von Poly(oxyalkylen)acrylamid, wobei man ein aliphatisches Poly(oxyalkylen)amin in Gegenwart einer Hydrolase in Substanz oder in einem ein organisches Lösungsmittel umfassenden, flüssigen Reaktionsmedium mit einer Acrylsäureverbindung oder einem Alkylester davon umsetzt, und man das (die) gebildete(n) Poly(oxyalkylen)acrylamid(e) nach Beendigung der Reaktion gegebenenfalls aus dem Reaktionsgemisch isoliert.

2. Verfahren nach Anspruch 1, wobei das flüssige Reaktionsmedium einen anfänglichen Wassergehalt von weniger als etwa 10 Vol.-% aufweist.

3. Verfahren nach einem der vorhergehenden Ansprüche, wobei Acrylsäureverbindung und Poly(oxyalkylen)amin in einem molaren Verhältnis von etwa 100:1 bis 1:1 eingesetzt werden.

4. Verfahren nach einem der vorhergehenden Ansprüche, wobei die Acrylsäureverbindung ausgewählt ist unter Acrylsäure, Niedrigalkyl-substituierter Acrylsäure, und den Alkylestern dieser Verbindungen, sowie Mischungen davon.

5. Verfahren nach einem der vorhergehenden Ansprüche, wobei die Hydrolase eine Lipase (E.C. 3.1.1.3.) ist.

6. Verfahren nach Anspruch 5, wobei das Poly(oxyalkylen)amin ausgewählt ist unter Poly(oxyalkylen)amin mit Molmassen von 500 bis 10000 g/mol

7. Verfahren nach einem der vorherigen Ansprüche, wobei der Enzymgehalt im Reaktionsmedium im Bereich von etwa 0,01 bis 10 Gew.-%, bezogen auf das eingesetzte Poly(oxyalkylen)amin liegt,

8. Verfahren nach einem der vorherigen Ansprüche, wobei die Reaktionstemperatur im Bereich von 0 bis etwa 100°C liegt.

9. Verfahren nach einem der vorherigen Ansprüche, wobei das Reaktionsmedium ein- oder mehrphasig ist und worin die Reaktanden gelöst, suspendiert oder emulgiert vorliegen.

10. Verfahren nach einem der vorherigen Anspruch, wobei man während der Reaktion anfallenden Alkohol oder Reaktionswasser aus dem Reaktionsgleichgewicht entfernt.

## Claims

1. A process for enzymatically synthesizing poly(oxyalkylene)acrylamide, wherein an aliphatic poly(oxyalkylene)amine is reacted in the presence of a hydrolase in bulk or in a liquid reaction medium comprising an organic solvent with an acrylic acid compound or an alkyl ester thereof, and the poly(oxyalkylene)acrylamide(s) formed is/are isolated if appropriate from the reaction mixture after the reaction has ended.

2. The process according to claim 1, wherein the liquid reaction medium has an initial water content of less than about 10% by volume.

3. The process according to either of the preceding claims, wherein acrylic acid compound and poly(oxyalkylene)amine are used in a molar ratio of from about 100:1 to 1:1.

4. The process according to any of the preceding claims, wherein the acrylic acid compound is selected from acrylic acid, lower alkyl-substituted acrylic acid and the alkyl esters of these compounds, and also mixtures thereof.

5. The process according to any of the preceding claims, wherein the hydrolase is a lipase (E.C. 3.1.1.3.).

6. The process according to claim 5, wherein the poly(oxyalkylene)amine is selected from poly(oxyalkylene)amine having molar masses of from 500 to 10 000 g/mol

7. The process according to any of the previous claims, wherein the enzyme content in the reaction mixture is in the range from about 0.01 to 10% by weight based on the poly(oxyalkylene)amine used.

8. The process according to any of the previous claims, wherein the reaction temperature is in the range from 0 to about 100°C.

9. The process according to any of the previous claims, wherein the reaction medium is monophasic or multiphasic and the reactants are dissolved, suspended or emulsified therein.

10. The process according to any of the previous claims, wherein alcohol or water of reaction formed during the reaction is removed from the reaction equilibrium.

## Revendications

1. Procédé pour la synthèse enzymatique de poly(oxyalkylène)acrylamide, où on transforme une poly(oxyalkylène)amine aliphatique en présence d'une hydrolase en masse ou dans milieu réactionnel liquide comprenant un solvant organique avec un composé d'acide acrylique ou un ester d'alkyle de celui-ci et on isole le cas échéant le/les poly(oxyalkylène)acrylamide(s) formé(s) à la fin de la réaction du mélange réactionnel.

2. Procédé selon la revendication 1, où le mélange réactionnel liquide présente une teneur en eau de départ inférieure à 10% en volume.

3. Procédé selon l'une quelconque des revendications précédentes, où le composé d'acide acrylique et la poly(oxyalkylène)amine sont utilisés dans un rapport molaire d'environ 100:1 à 1:1.

4. Procédé selon l'une quelconque des revendications précédentes, où le composé d'acide acrylique est choisi parmi l'acide acrylique, l'acide acrylique substitué par un alkyle inférieur et les esters d'alkyle de ces composés ainsi que leurs mélanges.

5. Procédé selon l'une quelconque des revendications précédentes, l'hydrolase étant une lipase (E.C. 3.1.1.3.).

6. Procédé selon la revendication 5, où la poly(oxyalkylène)amine est choisie parmi les poly(oxyalkylène)amines présentant des masses molaires de 500 à 10 000 g/mole.

7. Procédé selon l'une quelconque des revendications précédentes, où la teneur en enzyme dans le milieu réactionnel se situe dans la plage d'environ 0,01 à 10% en poids, par rapport à la poly(oxyalkylène)amine utilisée.

8. Procédé selon l'une quelconque des revendications précédentes, dans lequel la température de réaction se situe dans la plage de 0 à environ 100°C.

9. Procédé selon l'une quelconque des revendications précédentes, où le milieu réactionnel est à une ou plusieurs phases et dans lequel les réactifs se trouvent sous forme dissoute, en suspension ou en émulsion.

10. Procédé selon l'une quelconque des revendications précédentes, où on élimine l'alcool ou l'eau de réaction produit(e) pendant la réaction de l'équilibre de réaction.
